# FASCICULE DE BREVET EUROPEEN

(11) **EP 4 489 908 B1**
(45) Date de publication et mention de la délivrance du brevet: **21.01.2026**
(21) Numéro de dépôt: 23709730.8
(22) Date de dépôt: 10.03.2023
(51) Int. Cl.: B01J 35/58, B01J 35/39, B01J 21/06, A61L 9/20, D01F 8/06, D01F 1/10

(54) **PROCEDE DE FABRICATION D'UNE FIBRE MULTI-COMPOSANTE PHOTOCATALYTIQUE ET FIBRE MULTI-COMPOSANTE PHOTOCATALYTIQUE**
VERFAHREN ZUR HERSTELLUNG EINER PHOTOKATALYTISCHEN MEHRKOMPONENTENFASER UND PHOTOKATALYTISCHE MEHRKOMPONENTENFASER
METHOD FOR MANUFACTURING A PHOTOCATALYTIC MULTICOMPONENT FIBER, AND PHOTOCATALYTIC MULTICOMPONENT FIBER

(30) Priorité: 11.03.2022 FR 2202158
(43) Date de publication de la demande: 15.01.2025
(73) Titulaire: PURENAT, 64600 Anglet (FR)
(72) Inventeur: KINADJIAN CAPLAT, Natacha, 64600 Anglet (FR)
(74) Mandataire: A.P.I. Conseil
(86) Numéro de dépôt international: PCT/EP2023/056191
(87) Numéro de publication internationale: WO 2023/170275

(56) Documents cités:
- CN-A- 104 499 088
- DE-A1- 102004 021 425
- JP-A- H 101 879
- JP-B2- 3 713 122

## Description

### Domaine technique

L'invention concerne le domaine du textile. En particulier, l'invention concerne le domaine des fibres textiles.

L'invention concerne plus précisément un procédé de fabrication d'une fibre multi-composante photocatalytique ainsi qu'une fibre multi-composante photocatalytique en tant que telle. En outre, l'invention concerne un textile comportant une fibre multi-composante photocatalytique ainsi qu'un filtre doté d'au moins un textile selon l'invention.

### Technique antérieure

Ci-après, nous décrivons l'art antérieur connu à partir duquel l'invention a été développée.

La pollution de l'air et de l'eau n'a cessé d'augmenter ces dernières années. La présence de molécules organiques ou inorganiques dans l'air et/ou l'eau est surveillée de façon assidue. En effet, il a été prouvé que la pollution de l'air entraine d'une part de nombreuses pathologies (cardiorespiratoire ou encore certains cancers par exemple) mais également une détérioration et une pollution aggravées de l'environnement. De même, la pollution des eaux (nappe phréatique, lac, rivière, mer et océan) ne cesse de croître au détriment de la faune et de la flore. Aussi, de nombreux appareils de purification (de l'air et/ ou de l'eau) se sont développés. Ces appareils se destinent en particulier à l'élimination de polluants, pathogènes ou encore allergènes.

Ces appareils de purification fonctionnent généralement par un procédé physique par exemple par filtre ou dépoussiéreur électrostatique, ou encore par absorption des polluants sur des matériaux (comme les charbons actifs par exemple), ou par destruction ou inactivation à l'aide de rayons ultraviolets ou par photocatalyse. Certains systèmes combinent plus d'une technologie et chaque appareil de purification peut présenter des avantages et des risques différents.

Dans le domaine du traitement de l'air, les photocatalyseurs sont des matériaux qui peuvent décomposer la matière sans émission de polluants secondaires. Le TiO₂ est l'un des photocatalyseurs les plus utilisé. C'est un matériau semi-conducteur qui permet diverses réactions redox dont notamment la décomposition des polluants en utilisant les caractéristiques liées à l'absorption de la lumière et plus précisément dans le domaine de l'UV. De plus, le TiO₂ outre sa forte photoactivité, est un matériau stable qui présente des rendements élevés et qui de façon particulièrement avantageuse pour l'industrie est peu coûteux.

Le procédé de traitement mis en œuvre par les purificateurs photocatalytiques permet la décomposition et la dégradation de polluants sous l'action de rayons lumineux à la surface d'un photocatalyseur, généralement du dioxyde de titane (TiO₂). Le procédé permet de détruire les composés organiques volatils, les polluants inorganiques et les microorganismes. Le processus finalisé produit essentiellement de l'eau et du dioxyde de carbone.

Pour les purificateurs photocatalytiques, le photocatalyseur est généralement déposé sur la surface d'un substrat. Le photocatalyseur peut être déposé sous forme de poudre, de suspension ou de solution. Ce type de dépôt à la surface d'un substrat peut entrainer la formation d'agrégats de photocatalyseur, et un recouvrement seulement partiel sur de faibles épaisseurs. Dans ce cadre, le photocatalyseur à tendance à se décrocher de son substrat facilement, ce qui rend inactif le substrat sur lequel il était déposé très rapidement.

En outre, il a été proposé des liants pouvant être ajoutés pour améliorer l'adhérence du TiO₂ au substrat. Toutefois, de tels liants peuvent diminuer drastiquement l'efficacité photocatalytique.

Enfin, l'imprégnation d'une surface avec un mélange comportant un photocatalyseur peut modifier fortement la dynamique des fluides au niveau de la surface imprégnée et diminuer drastiquement l'efficacité photocatalytique et augmenter la perte de charge dans le purificateur d'air.

Récemment, il a été proposé d'utiliser des fibres composites comportant des polymères et des faibles quantités de photocatalyseur pour former des filtres pour purificateurs d'air. Néanmoins avec ces techniques, le photocatalyseur est piégé dans la fibre photocatalytique de façon aléatoire ce qui réduit considérablement son efficacité. Ainsi une fibre peut comprendre des agrégats de TiO₂ ou à l'inverse des zones sans TiO₂. De plus, la présence d'agrégats dans le polymère peut entraver les étapes ultérieures de fabrication comme le filage lorsque le substrat est sous forme de fibres.

En outre, actuellement la plupart des substrats pour fixer le TiO₂ comprennent des fibres issues de fibres de verre, de céramique, de minéral argileux, zéolite, plaque métallique, cellulose ou encore de charbon actif. Or, ces substrats sont d'une part particulièrement fragiles et d'autre part très peu flexibles ce qui entraine une limitation dans la géométrie des purificateurs qui se ressemblent tous à l'heure actuelle. En effet, lors du filage, les fibres sont étirées, chauffées et refroidies à plusieurs reprises. Les fibres peuvent alors subir des dommages tels que des fissures voir des ruptures. En particulier ces fibres peuvent être étirées à outrance et subir une perte de masse, une perte de charge voire une chute de force. Ceci limite grandement la mise en forme et la géométrie des textiles et donc des photocatalyseurs.

Par ailleurs, lors d'une industrialisation, il devient très difficile d'assurer une bonne capacité et efficacité d'un photocatalyseur basé sur des fibres contenant du TiO₂. D'une part afin d'assurer de bonnes performances photocatalytiques, il faut intégrer dans la fibre une quantité suffisante de photocatalyseur tout en assurant une fluidité suffisante de la matrice lors du filage et une répartition homogène du TiO₂, et d'autre part, il est nécessaire d'assurer un filage optimal de sorte que les fibres préservent leurs caractéristiques mécaniques et leur maniabilité tout en limitant la perte de charge.

Afin de remédier aux problèmes d'agrégats et d'assurer une dispersion optimale du TiO₂ il a par exemple été proposé dans le document US2020282387 un polymère d'oxyde de titane linéaire. Dans ce document, le revêtement est formé par frittage d'une solution comprenant le polymère pouvant être déposée sur un substrat sous forme de fibres. Néanmoins, dans ce document, le substrat est de préférence rugueux avec des surfaces externes en protubérances. Ceci permet d'améliorer l'adhérence du TiO₂ au substrat mais accentue la formation d'agrégats de TiO₂ en surface. En outre, le frittage confère une forte rigidité à la structure, ce qui réduit la maniabilité et empêche toute mise en forme. De plus, un tel produit est très friable et très fragile ce qui limite notamment la réduction de son épaisseur pour réduire la perte de charge.

D'autres techniques ont été développées comme par exemple un procédé de fabrication de fibres macroscopiques de TiO₂ par extrusion en continu en flux unidirectionnel décrit dans le document EP3126550. Un tel procédé permet de produire à grande échelle des fibres macroscopiques de TiO₂. Cependant, afin de pouvoir obtenir des bonnes performances photocatalytiques, le polymère est entièrement calciné après la fabrication des fibres qui ne contiennent alors plus que du TiO₂ et sont extrêmement cassantes. Leur résistance mécanique après calcination ne permet pas d'appliquer un flux d'air assez puissant pour son utilisation dans un purificateur d'air.

Le document JP-B2-3 713122 divulgue un procédé de fabrication d'une fibre du type cœur-écorce photocatalytique. La fibre est basée sur un polyester thermoplastique et contient 0.5-10 % en poids de TiO2. L'écorce est enrichie en TiO2 et traité après le filage avec un plasma du type corona pour éliminer le polymère en surface et activer le TiO2.

L'invention a pour but de remédier aux inconvénients de l'art antérieur. En particulier, l'invention a pour but de proposer un procédé de fabrication d'une fibre multi-composante à forte concentration surfacique de photocatalyseur, présentant une répartition homogène du photocatalyseur de sorte à optimiser l'efficacité d'un purificateur d'air photocatalytique utilisant cette fibre, tout en permettant de proposer un tissu malléable pouvant supporter différentes mises en forme tout en conservant une forte perméabilité à l'air.

### Résumé de l'invention

L'invention vise à pallier ces inconvénients.

L'invention vise en particulier un procédé de fabrication d'une fibre multi-composante photocatalytique comportant les étapes suivantes :
- Fournir un mélange support, ledit mélange support comportant au moins un polymère thermoplastique ou un précurseur de polymère thermoplastique ;
- Fournir un mélange actif, ledit mélange actif comportant :
   ∘ au moins un polymère organique ou un précurseur de polymère organique,
   ∘ au moins un photocatalyseur à une concentration d'au moins 10 % en poids par rapport au poids de mélange actif,
   ∘ au moins un agent couplant résistant à l'oxydation, de préférence un silane ; ou un précurseur d'agent couplant résistant à l'oxydation,
- Filage d'une fibre multi-composante à partir des mélanges support et actif ;
- Elimination de l'au moins un polymère organique en surface de la fibre multi-composante de façon à générer une fibre multi-composante photocatalytique.

La demanderesse a développé un procédé capable de générer une fibre multi-composante photocatalytique présentant un taux de photocatalyseur élevé, une répartition en surface du photocatalyseur homogène tout en étant déformable et présentant des caractéristiques mécaniques compatibles avec leur utilisation dans un purificateur d'air. En effet, l'étape d'élimination permet de former une surface inorganique, de préférence majoritairement inorganique, comportant le photocatalyseur au contact d'un support polymérique thermoplastique.

La demanderesse a développé en particulier un mélange support et un mélange actif qui combinés permettent d'assurer la production d'une fibre flexible présentant une forte teneur du photocatalyseur en surface et une répartition homogène en surface du photocatalyseur de sorte que les agrégats de photocatalyseur soient réduits ou inexistants, et que la surface de la fibre soit majoritairement recouverte de photocatalyseur.

En outre, un tel procédé donne également la possibilité de filer des fibres sans rupture, fissure, ou encore chute de force.

Ainsi, un procédé selon l'invention permet de répondre aux besoins et notamment de proposer la génération d'une fibre multi-composante photocatalytique à forte concentration surfacique de photocatalyseur de sorte à optimiser la capacité et l'efficacité du photocatalyseur, et sa répartition homogène procurant une surface de fibre multi-composante poreuse avec peu ou pas d'agrégats tout en étant malléable afin de pouvoir répondre à différentes mises en forme.

Selon d'autres caractéristiques optionnelles du procédé, ce dernier peut inclure facultativement une ou plusieurs des caractéristiques suivantes, seules ou en combinaison :
- l'élimination de l'au moins un polymère organique en surface de la fibre multi-composante comprend une calcination de surface de façon à générer une surface inorganique au contact d'un support polymérique thermoplastique. Ceci permet d'une part d'activer le photocatalyseur et d'autre part d'assurer une répartition homogène, tout en assurant une surface poreuse à la fibre multi-composante photocatalytique pour améliorer les échanges avec l'air ;
- le ou les polymères thermoplastiques du mélange support sont sélectionnés parmi les polymères thermoplastiques présentant une température de fusion comprise entre 100 °C et 350 °C. Ces polymères thermoplastiques du mélange support sont de préférence biosourcés et/ou biodégradables ;
- il comporte une extrusion et/ou une coextrusion du mélange support et/ou du mélange actif ;
- le mélange support et le mélange actif présentent des indices de fluidité à chaud mesurés selon la norme ISO 1133, tels que l'écart entre l'indice de fluidité à chaud du mélange actif et l'indice de fluidité à chaud du mélange support est inférieur ou égal à 20 %. Ceci permet un meilleur contrôle de la viscosité des mélanges support et actif afin d'assurer un filage optimal sans rupture, et/ou chute de force. En outre, cela peut permettre d'assurer des propriétés mécaniques optimisées à la fibre multi-composante photocatalytique,
- lors du filage la fibre multi-composante présente un taux de gaine compris entre 5 % et 50 % calculé selon la formule 4*e* * (*D - e*)/*D*². Ce qui peut assurer une répartition homogène du photocatalyseur et réduit l'effet collier de perle.

Selon un deuxième objet, l'invention porte sur **une fibre multi-composante** photocatalytique comportant :
- un support polymérique thermoplastique, et
- Un mélange actif correspondant à une surface inorganique ;
le support polymérique thermoplastique comportant au moins un polymère thermoplastique, et la surface inorganique comportant un réseau d'agent couplant, résistant à l'oxydation, associé à un photocatalyseur, la fibre multi-composante photocatlytique combinant le mélange support et le mélange actif filé.

Selon d'autres caractéristiques optionnelles de la fibre multi-composante photocatalytique, cette dernière peut inclure facultativement une ou plusieurs des caractéristiques suivantes, seules ou en combinaison :
- elle présente un diamètre inférieur ou égal à 150 µm,
- la surface inorganique présente une épaisseur d'au moins 500 nm,

Selon un troisième objet, l'invention porte sur **un textile** comportant au moins une fibre multi-composante photocatalytique selon l'invention. Un tel textile selon l'invention est flexible et malléable.

Selon un quatrième objet, l'invention porte sur **un filtre** comportant au moins un textile selon l'invention. Un tel filtre peut présenter des géométries variables par exemple plissé contrairement aux filtres existants. En outre, un filtre selon l'invention peut être de conformation adaptée par exemple sur-mesure contrairement aux filtres existants.

Selon un cinquième objet, l'invention porte sur un **épurateur d'air photocatalytique** doté d'au moins un filtre selon l'invention, d'un système de ventilation et d'un système d'illumination ultraviolet apte à illuminer l'au moins un filtre ; ledit ventilateur étant agencé de façon à véhiculer de l'air depuis une entrée du système d'épuration à une sortie du système d'épuration à travers le filtre.

### Brève description des dessins

D'autres caractéristiques et avantages de l'invention seront mieux compris à la lecture de la description qui va suivre et en référence aux dessins annexés, donnés à titre illustratif et nullement limitatif.
[Fig. 1] La figure 1 représente un diagramme d'un procédé de fabrication d'une fibre multi-composante photocatalytique selon un mode de réalisation de l'invention.
[Fig. 2] La figure 2 représente un schéma présentant différentes section de fibres multi-composantes photocatalytiques selon l'invention.
[Fig.3] La figure 3 représente un cliché d'une fibre multi-composante photocatalytique selon un mode de réalisation de l'invention, selon un grossissement x80 par microscopie électronique à balayage (MEB).

Les figures ne respectent pas nécessairement les échelles, notamment en épaisseur, et ce à des fins d'illustration.

Des aspects de la présente invention sont décrits en référence à des organigrammes et / ou à des schémas fonctionnels de procédés et d'appareils (systèmes) selon des modes de réalisation de l'invention.

Sur les figures, les organigrammes et les schémas fonctionnels illustrent l'architecture, la fonctionnalité et le fonctionnement d'implémentations possibles de systèmes et de procédés selon divers modes de réalisation de la présente invention. Dans certaines implémentations, les fonctions associées aux blocs peuvent apparaître dans un ordre différent que celui indiqué sur les figures. Par exemple, deux blocs montrés successivement peuvent, en fait, être exécutés sensiblement simultanément, ou les blocs peuvent parfois être exécutés dans l'ordre inverse, en fonction de la fonctionnalité impliquée.

### Description des modes de réalisation

Ci-après, nous décrivons un résumé de l'invention et le vocabulaire associé, avant de présenter les inconvénients de l'art antérieur, puis enfin de montrer plus en détail comment l'invention y remédie.

Dans la suite de la description, l'expression « fibre multi-composante » peut correspondre à une fibre bi-composante, tri-composante ou plus.

Le terme « **mélange** support » au sens de l'invention peut correspondre à un mélange destiné à former un réseau en soutien au mélange actif de sorte que le mélange actif repose sur, autour ou dans le mélange support. Par exemple dans une configuration de fibre cœur-écorce, un mélange support peut correspondre à un cœur de fibre.

Le terme « **mélange** actif » au sens de l'invention peut correspondre à un mélange destiné à former un réseau de préférence inorganique et en surface du mélange support et destiné à interagir avec un stimulus physique lors de la réaction de photocatalyse. Par exemple, dans une configuration de fibre cœur-écorce, le mélange actif peut correspondre à l'écorce de fibre.

Le terme « cœur » au sens de l'invention peut correspondre à une partie interne d'une fibre telle qu'une âme.

Le terme « écorce » au sens de l'invention peut correspondre à une partie externe d'une fibre telle qu'une enveloppe.

L'expression « **polymère** thermoplastique » au sens de l'invention peut correspondre à un polymère qui, de manière répétée, peut être ramolli ou fondu sous l'action de la chaleur et qui adopte de nouvelles formes par application de chaleur et de pression.

L'expression « **précurseur de polymère** thermoplastique » au sens de l'invention peut correspondre à un composant permettant de commencer ou d'initier une réaction de polymérisation d'un ou plusieurs monomère(s).

L'expression « polymère organique » au sens de l'invention peut correspondre à un polymère, linéaire, ramifié ou cyclique, dont le noyau polymérique comprend au moins un atome de carbone.

Le terme **"polymérisation"** au sens de l'invention peut correspondre au processus permettant de convertir un monomère ou un mélange de monomères en polymère.

Le terme « **majoritairement** » au sens de l'invention peut correspondre à au moins 50 %, de préférence plus de 50 %. Par exemple une surface majoritairement inorganique au sens de l'invention peut correspondre à une surface comportant, en masse, plus de polymère inorganique que de polymère organique ou en mole, plus de molécule inorganique que de molécule organique. Une surface majoritairement inorganique peut par exemple comporter au moins 50 % en poids de photocatalyseur et d'agent couplant.

L'expression « **résistant à** l'oxydation » au sens de l'invention peut correspondre à une diminution ou à une limitation des interactions avec l'oxygène de sorte que les réactions d'oxydoréduction soient réduites particulièrement lors d'une photocatalyse.

L'expression « sensiblement **égal »** au sens de l'invention peut correspondre à une valeur variant de moins de 50 % par rapport à la valeur comparée, de préférence de moins de 40 %, de façon encore plus préférée de moins de 30 %. Lorsque sensiblement égal est utilisée pour comparer des valeurs alors la valeur comparée varie de moins de 50 % par rapport à la valeur prise en référence, de préférence de moins de 40 %, de façon encore plus préférée de moins de 30 %.

L'invention propose de prendre en considération les difficultés existantes associées à des filtres photocatalytiques peu maniables et en particulier pouvant entrainer une perte de charge et/ou la diminution de l'efficacité des photocatalyseurs.

En particulier, l'invention propose un procédé de fabrication d'une fibre multi-composante comprenant un support polymérique combiné à une surface inorganique, de préférence majoritairement inorganique. Un tel procédé permet de former une fibre avec une résistance mécanique élevée capable d'être façonnée sans l'endommager puis de former un filtre présentant une surface photocatalytique combinant forte perméabilité comparée à un textile imprégné présentant les mêmes caractéristiques (épaisseur, densité, taille de fibres) et haut taux de photocatalyseur.

Ainsi, l'invention concerne un procédé de fabrication d'une fibre multi-composante.

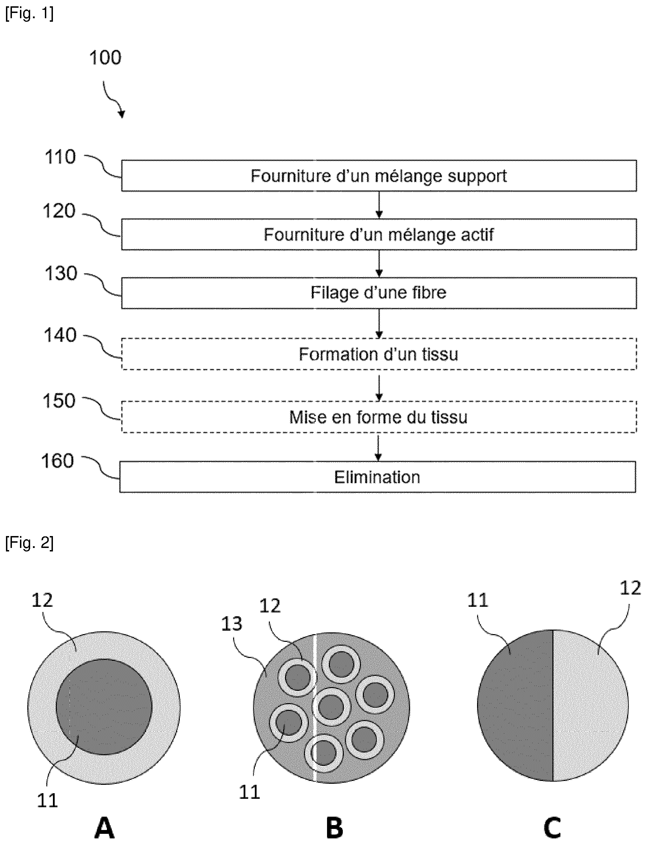

**La** **figure** 1 illustre un exemple de procédé 100 de fabrication d'une fibre multi-composante photocatalytique. Un tel procédé 100 de fabrication comprend une étape 110 de fourniture d'un mélange support, une étape 120 de fourniture d'un mélange actif, une étape 130 de filage, et une étape 160 d'élimination d'au moins un polymère organique en surface de la fibre multi-composante. En outre, un procédé 100 de fabrication selon l'invention peut comporter une étape de formation d'un tissu 140 et une étape de mise en forme 150 du tissu.

Dans l'exemple de la figure 1, le procédé 100 de fabrication d'une fibre multi-composante photocatalytique comprend **une étape de fourniture 110 d'un mélange support.** Le mélange support comporte au moins un polymère thermoplastique. Alternativement, le mélange support comporte au moins un précurseur de polymère thermoplastique. Selon une autre alternative le mélange support comporte au moins un polymère thermoplastique et au moins un précurseur de polymère thermoplastique.

Le ou les polymères thermoplastiques du mélange support peuvent être sélectionnés parmi les polymères thermoplastiques présentant une température de fusion inférieure à 350 °C, par exemple une température de fusion comprise entre 100 °C et 350 °C. Avantageusement, le ou les polymères thermoplastiques du mélange support peuvent être sélectionnés parmi : polypropylène, polyester, polyéthylène, acide polylactique, polyamide, polyvinyle, polyacrylate, polytéréphtalate de butylène, polyhydroxyalkanoates, poly(butylène adipate-co-terephthalate et leur mélange. Par exemple, le ou les polymères thermoplastiques du mélange support peuvent être sélectionnés parmi : polyéthylène téréphtalate, bio- polyéthylène téréphtalate, bio-polyéthylène, polyester biodégradables, et polyhydroxyalcanoate.

De préférence, le ou les polymères thermoplastiques du mélange support peuvent être sélectionnés parmi les polymères thermoplastiques biosourcés et/ou biodégradables.

Un précurseur de polymère thermoplastique selon l'invention pour le mélange support peut par exemple être sélectionné parmi tous précurseurs de polymère thermoplastique présentant une température de fusion comprise entre 100 °C et 350 °C. Avantageusement, le ou les précurseurs de polymère thermoplastique peuvent être sélectionnés parmi lactides, acrylates, méthacrylates, styrènes, et/ou lactones.

Avantageusement, le ou les polymères thermoplastiques du mélange support et/ou le ou les polymères thermoplastiques formés à partir du précurseur de polymère thermoplastique du mélange support présentent de bonnes propriétés pour le filage.

Une étape de fourniture d'un mélange support peut par exemple comprendre la préparation d'un mélange support. Dans ce mode de réalisation, lorsque le mélange support comprend un précurseur tel qu'au moins un monomère de polymère thermoplastique, l'étape de préparation du mélange support peut comprendre une étape de polymérisation. Une étape de polymérisation peut être réalisée à l'aide d'un stimulus tels qu'un plasma, un bombardement ionique, un processus électrochimique, une espèce chimique (nucléophile, électrophile...), un rayonnement lumineux. Par ailleurs, une étape de polymérisation peut être réalisée selon une durée et une température prédéterminée en fonction de l'au moins un monomère.

L'étape de préparation d'un mélange support peut être réalisée à une température inférieure ou égale à 400 °C et supérieure ou égale à 100 °C.

Optionnellement, l'étape de préparation du mélange support peut comprendre l'ajout d'additif, par exemple pour améliorer la résistance du support flexible. Par exemple un cœur de la fibre multi-composante photocatalytique dans un mode de configuration de fibre cœur-écorce.

Le procédé 100 de fabrication d'une fibre multi-composante photocatalytique comprend **une étape de fourniture 120 d'un mélange actif.**

Le mélange actif comporte **au moins un polymère organique,** comme par exemple au moins un polymère thermoplastique. Alternativement, le mélange actif comporte au moins un précurseur de polymère organique. Selon une autre alternative le mélange actif comporte au moins un polymère organique et au moins un précurseur de polymère organique.

Le ou les polymères du mélange actif peuvent être sélectionnés parmi les polymères présentant une température de fusion comprise entre 100 °C et 350 °C. Avantageusement le ou les polymères du mélange actif peuvent être sélectionnés parmi : polypropylène, polyester, polyéthylène, acide polylactique, polyamide, polyvinyle, polyacrylate, polytéréphtalate de butylène, polyhydroxyalkanoates, poly(butylène adipate-co-terephthalate et leur mélange. Par exemple, le ou les polymères du mélange actif peuvent être sélectionnés parmi : polyéthylène téréphtalate, bio- polyéthylène téréphtalate, bio-polyéthylène, polyester biodégradables, et polyhydroxyalcanoate.

De préférence, le ou les polymères du mélange actif peuvent être sélectionnés parmi les polymères thermoplastiques biosourcés et/ou biodégradables.

Par ailleurs, le ou les polymères du mélange actif peuvent comprendre un groupement chimique supplémentaire greffé.

Un précurseur de polymère du mélange actif selon l'invention peut par exemple être sélectionné parmi tous précurseurs de polymère présentant une température de fusion comprise entre 100 °C et 350 °C. Avantageusement, le ou les précurseurs de polymère peuvent être sélectionné parmi lactides, acrylates, méthacrylates, styrènes, et lactones.

Dans un mode de réalisation particulier de l'invention, la fourniture du mélange actif peut comprendre une étape de préparation d'un mélange actif.

Une étape de préparation d'un mélange actif peut être réalisée à une température inférieure ou égale à 400 °C et supérieure ou égale à 100 °C.

Dans le cas où la préparation du mélange actif comprend au moins un monomère de polymère thermoplastique, l'étape de préparation du mélange actif peut comprendre une étape de polymérisation. Une étape de polymérisation peut être réalisée à l'aide d'un stimulus tels qu'un plasma, un bombardement ionique, un processus électrochimique, une espèce chimique (nucléophile, électrophile...), un rayonnement lumineux. Par ailleurs, une étape de polymérisation peut être réalisée selon une durée et une température prédéterminée en fonction de l'au moins un monomère.

Dans un mode de réalisation particulier de l'invention, le mélange actif comporte les mêmes polymères ou précurseur de polymère que le mélange support.

Avantageusement, le ou les polymères, de préférence thermoplastiques, du mélange actif et/ou le ou les polymères formés à partir du précurseur de polymère, de préférence thermoplastique, du mélange actif présentent de bonnes propriétés pour le filage.

Le mélange actif comprend **au moins un photocatalyseur.** De préférence, le mélange actif comprend au moins un photocatalyseur à une concentration d'au moins 10 % en poids par rapport au poids de mélange actif, de façon préférée au moins 15 %, au moins 20 %, au moins 25 %, au moins 30 %, au moins 35 %, au moins 40 %. De préférence, le mélange actif comprend un photocatalyseur à une concentration inférieure ou égale à 50 % en poids par rapport au poids du mélange actif, de préférence à une concentration inférieure ou égale à 45 %. Par exemple, le ou les photocatalyseurs seront présents dans le mélange actif à une concentration de 10 % à 50 % en poids par rapport au poids de mélange actif, de façon préférée à une concentration de 15 % à 45 % en poids par rapport au poids de mélange actif, de façon plus préférée de 20 % à 45 % en poids par rapport au poids de mélange actif et de façon encore plus préférée à une concentration de 25 % à 40 %. Le photocatalyseur peut être dosé par doseur gravimétrique.

Un photocatalyseur au sens de l'invention peut être sélectionné parmi : métaux de transition, métaux pauvres, metalloïdes et leur oxyde, de préférence ayant des propriétés de photocatalyse. Par exemple un photocatalyseur peut être choisi parmi AgBr, AgCl, Ag₃PO₄, Ag₂S , Agl, Bi₂O₃, Bi₂S₃, C₃N₄, CdS, CdSe, CdO, Ce₂0₃, Ce₂S₃ CoO, CuO, Cu₂O, Cu₂S, CuInS₂, FeTi0₃, Fe₂O₃, GaAs, GaP, In₂S₃, MoS₂, Nn₂0₃, NiO, PbO, PdO, RuO₂, SnO2, SnS, TiO₂, V₂O₅, WS₂, WO₃, ZnO, ZnS, ZrS₂ ZnSe, ZrO₂ et leur mélange.

Le photocatalyseur peut éventuellement être dopé et/ou greffé. Par exemple, un photocatalyseur peut être prétraité par un traitement hydrophobe.

De préférence, le photocatalyseur est le TiO₂.

Le photocatalyseur peut se présenter sous forme cristalline. Dans le cas particulier du TiO₂, celui-ci peut être sous forme d'anatase ou de mélange anatase et rutile ou de mélange anatase, rutile et brookite.

Le photocatalyseur peut se présenter sous forme de nanoparticules présentant un diamètre moyen de 2 nm à 100 nm, de préférence de 5 nm à 75 nm, de manière plus préférée entre 10 nm et 50 nm. Le photocatalyseur peut se présenter sous forme de poudre ou sous la forme d'un précurseur en solution.

Selon un mode de réalisation, le photocatalyseur est incorporé au mélange actif lorsqu'il est à l'état fondu. Cela permet de limiter et de minimiser la formation d'agrégat.

Le mélange actif comprend **au moins un agent couplant ou un précurseur d'agent couplant.** De préférence, un agent couplant selon l'invention résistant à l'oxydation.

Un agent couplant peut comprendre une fonction chimique permettant de réaliser une liaison chimique avec au moins un photocatalyseur, de préférence avec le TiO₂. Un agent couplant peut comprendre une fonction chimique permettant une réaction de polymérisation de l'agent couplant de sorte à former un réseau. Un agent couplant peut comprendre une fonction chimique apte à réaliser une liaison chimique avec un polymère thermoplastique du mélange support.

Un agent couplant peut être sélectionné parmi un ou plusieurs géopolymères ou un ou plusieurs précurseurs de géopolymères.

Un agent couplant peut être sélectionné parmi les silanes, ou les siloxanes par exemple.

De préférence l'agent couplant est sélectionné parmi : vinyltrimethoxysilane, polydiméthylsiloxane, tétraéthoxysilane, tétraméthoxysilane, tétrapropoxysilane n-propyltriethoxysilane, éthyltriméthoxysilane, méthyltriéthoxysilane, propyltriméthoxysilane , propyltriéthoxysilane, phényltriméthoxysilane, phényltriéthoxysilane , trimethoxyvinylsilane, triethoxyvinylsilane, vinyltriéthoxysilane, vinyltris(β-methoxyethoxy)silane, β-(3,4-epoxycyclohexyl)ethyltrimethoxysilane, γ-(2-aziridine) aminopropyltrimethoxysilane, γ-glycidoxypropyltrimethoxysilane, γ-glycidoxypropyltrimethyldiethoxysilane, γ-glycidoxypropyltriethoxysilane, γ-methylpropenylpropyldimethoxysilane, γ-methylpropenyltrimethoxysilane, γ-methylpropenylpropyldiethoxysilane, γ-methylpropenylpropyltriethoxysilane, N-β(aziridine)γ-aminopropylmethyldimethoxysilane, N-β(aziridine)γ-aminopropyltrimethoxysilane, N-β(aziridine)γ-aminopropyltriethoxysilane, N-β-(aminoethyl)-γ-aminopropyltriméthoxysilane 3-acryloxypropyltrimethoxysilane, 3-aminopropyltrimethoxysilane, 3-aminopropyltriethoxysilane, N-phenyl-γ-aminopropyltrimethoxysilane, γ-chloropropyltrimethoxysilane, γ-hydrothiopropyltrimethoxysilane, bis[3-(triethoxysilyl)propyl] tetrasulfide (TESPT) and bis[3-(triethoxysilyl)propyl]-disulfide, and preferably is 3-acryloxypropyltrimethoxysilane, 3-chloropropyltriméthoxysilane, 3-mercaptopropyltriméthoxysilane, 3-méthacryloxypropyltriméthoxysilane, 3-acryloxypropyltriméthoxysilane.et leur combinaison. Ces agents couplants permettent une bonne dispersion des charges et de simplifier le process de compoundage. De préférence, un agent couplant permet de former un réseau qui est inorganique, ce qui permet de résister à la photocatalyse. De préférence le réseau inorganique est réticulé. Il comporte de préférence des chaines polymériques reliées entre elles par des liaisons de plus faible masse moléculaires. Il peut alors former un réseau tri dimensionnel. De façon avantageuse, un réseau inorganique permet de résister à l'oxydation et donc à la photocatalyse.

Un précurseur d'agent couplant peut être sélectionné parmi : silice, siloxane, silanol.

Le mélange actif comprend au moins un agent couplant à une concentration d'au moins 0,75% en poids par rapport au poids de mélange actif, de façon préférée au moins 1,5 %, au moins 2 %, au moins 2,5 % au moins 3%. De préférence le mélange actif comprend un agent couplant à une concentration inférieure ou égale à 10% en poids par rapport au poids du mélange actif, de préférence à une concentration inférieure ou égale à 8%. Par exemple, le mélange actif comprend au moins un agent couplant à une concentration de 0,75% à 10 % en poids par rapport au poids de mélange actif, de façon préférée au moins 1,5 % et 8 % en poids par rapport au poids de mélange actif.

Avantageusement, l'ajout d'un agent couplant au mélange actif permet de diminuer la viscosité du mélange actif par rapport à la viscosité du même mélange actif sans agent couplant, mesuré par rhéomètre selon la norme ISO 1628 .

Le mélange actif peut comporter un rapport massique de l'au moins un agent couplant sur l'au moins un photocatalyseur compris entre 1/50 et 5/1. De préférence, le mélange actif peut comporter un rapport massique de l'au moins un agent couplant sur l'au moins un photocatalyseur compris entre 1/20 et 2/1. De façon plus préférée, le mélange actif peut comporter un rapport massique de l'au moins un agent couplant sur l'au moins un photocatalyseur compris entre 1/10 et 1.

Selon un mode de réalisation, l'agent couplant est incorporé au mélange actif au moyen d'une pompe par exemple une pompe péristaltique, par injection.

De façon préférée, le photocatalyseur utilisé dans le cadre de l'invention est préalablement combiné à l'agent couplant. Ainsi, selon un mode de réalisation préféré de l'invention, l'agent couplant est ajouté au mélange actif lorsqu'il est couplé au photocatalyseur. Ainsi, dans ce cas, le procédé peut comporter une étape de couplage du photocatalyseur à l'agent couplant ou une étape de fourniture d'un photocatalyseur couplé à l'agent couplant.

L'étape de fourniture d'un mélange support et/ ou l'étape de fourniture d'un mélange actif peut être réalisée par un mélangeur (compoundeur), un malaxeur, une extrudeuse et/ou un doseur de type doseur gravimétrique et/ou doseur volumétrique.

Dans un mode de réalisation préféré, l'étape de fourniture d'un mélange support et/ou l'étape de fourniture d'un mélange actif comprend une extrusion et/ou une et/ou une coextrusion. L'étape de fourniture d'un mélange support et/ou l'étape de fourniture d'un mélange actif peut comprendre une multiple extrusion. Par multiple il peut s'agir de supérieur ou égal à deux. Une multiple extrusion permet d'améliorer la dispersion du photocatalyseur dans le mélange actif ainsi que l'incorporation de l'agent couplant.

Lorsque l'étape de fourniture d'un mélange support comprend une extrusion du mélange support, l'étape d'extrusion est configurée de façon à ce que le mélange support présente un indice de fluidité à chaud (MFI) sensiblement égal à l'indice de fluidité à chaud du mélange actif, mesuré selon la norme ISO 1133 (tel que mesuré à 230°C pour une masse standard de 2160 g). Par exemple, le mélange support présente un indice de fluidité à chaud (MFI) dont l'écart avec l'indice de fluidité à chaud du mélange actif est inférieur ou égal à 20%, de préférence inférieur ou égale à 10% et de manière encore plus préférée inférieur ou égale à 5 %. Ainsi la différence entre le MFI du mélange support et le MFI du mélange actif est faible et de préférence inférieure ou égale à 15 %.

L'indice de fluidité à chaud du mélange actif peut par exemple être compris entre 5 g/10min et 1 500 g/10min mesuré selon la norme ISO 1133. Par exemple, l'indice de fluidité à chaud du mélange support peut être compris entre 9 g/10min et 15 g/10min. Dans un mode de réalisation, le MFI du mélange support peut être de 12 g/10min et le MFI du mélange actif peut être de 27 g/10min. Dans un autre mode de réalisation, le MFI du mélange support peut être de 12 g/10min et le MFI du mélange actif peut être de 5 g/10min. Dans un autre mode de réalisation, le MFI du mélange support peut être de 12 g/10min et le MFI du mélange actif peut être de 11 g/10min.

L'étape de préparation d'un mélange actif peut être réalisée à une température inférieure ou égale à 400 °C et supérieure ou égale à 100 °C, de préférence inférieure ou égale à 300 °C de manière plus préférée inférieure ou égale à 250 °C.

Un procédé 100 de fabrication d'une fibre multi-composante photocatalytique peut comporter **une étape de filage 130.** L'étape de filage est de préférence réalisée à partir d'un mélange support et d'un mélange actif.

Une étape de filage peut être réalisée à chaud par melt spinning (pour trempe sur roue), spun bond (pour étirer à froid) et/ou meltblown (pour étirer à chaud).

La **figure** 2 présente des exemples de configurations de filage selon la présente invention. Cette figure présente une partie des configurations possibles avec une configuration cœur-écorce (« Sheath-Core » en terminologie anglosaxonne) en figure 2A ; une configuration de type îles dans la mer (« island in the sea » en terminologie anglosaxonne) en figure 2B ; une configuration de type côte à côte (« side by side » en terminologie anglosaxonne) en figure 2C.

Les configurations illustrées aux figures 2A et 2B permettent de former une ou plusieurs fibres comportant un support en polymère thermoplastique 11 de type cœur et une surface inorganique 12 de préférence majoritairement inorganique, de type écorce inorganique. Comme cela est illustré à la figure 2B, la fibre multi-composantes peut comporter trois phases : un support en polymère thermoplastique 11, une surface inorganique 12, de préférence majoritairement inorganique et une matrice sacrificielle 13. En effet, dans un mode de réalisation particulier, la fibre multi-composante peut comporter une matrice sacrificielle supprimée généralement avant l'étape d'élimination 160 de l'au moins un polymère organique.

Comme illustré à la figure 2C, la fibre multi-composante peut comporter un support 11 au contact d'une surface inorganique 12, de préférence majoritairement inorganique.

Une étape d'étirement du fil supplémentaire peut être réalisée à froid et/ou à chaud afin de réduire le diamètre du fil obtenu.

De préférence l'étape de filage peut être réalisée afin d'atteindre un diamètre de fibre compris entre 2 µm et 150 µm.

Afin d'éviter des dommages tels que des fissures, des ruptures ou encore une perte de masse voire une chute de force, l'étape de filage est réalisée de sorte que la fibre présente un taux de gaine compris entre 5 et 50 %, de préférence entre 7 et 30% mesuré selon la formule *4e* * *(D - e*)/*D*² par microscopie (MEB). Avec un pourcentage de gaine égal à la surface de la gaine (Sg) divisé par la surface totale (St), avec Sg = St - Scoeur, soit Sg = π*D²/4-π*(D-2e)²/4 soit Sg = π*(4De-4e²)/4, soit Sg=π*e*(D-e) et donc le pourcentage de gaine correspond à 4e * *(D - e*)/*D*² . Avec D le diamètre extérieur de la gaine et e l'épaisseur de la gaine. Grâce à un taux de gaine maîtrisé, l'effet collier de perle (répartition inhomogène du photocatalyseur) est minimisé.

Avantageusement, l'étape de filage peut être réalisée par voie fondue et peut comprendre un étirement à chaud qui peut être réalisé au moyen d'au moins une extrudeuse comprenant une ou plusieurs zone de chauffe. De préférence, l'étape de filage par voie fondue comprend une extrudeuse pour le mélange support et une extrudeuse pour le mélange actif.

L'étape de filage peut être configurée pour faire varier le ratio (e.g. massique) de mélange support par rapport au mélange actif. Cela peut permettre de diminuer le diamètre de la fibre et par exemple de faire varier le ratio de cœur par rapport au ratio d'écorce pour une fibre de type cœur-écorce. En outre, la fibre obtenue grâce aux étapes de préparations du mélange support et du mélange actif présente une texture homogène (mesurée par microscopie MEB) lors de l'étape de filage qui témoigne d'une bonne dispersion des charges et d'une répartition homogène du photocatalyseur en surface. Les fibres peuvent être refroidies à l'air et directement bobinées.

L'étape de filage peut comprendre une étape d'étirement. Un étirement peut être réalisé, de préférence à une température comprise entre la température de transition vitreuse et la température de fusion des polymères des mélanges de support et/ou actif. Dans ce mode de réalisation, la bobine est dévidée et passée dans un premier banc de tirage à une vitesse V1, la fibre est réchauffée, de préférence entre 80 °C et 140 °C, et étirée à nouveau à l'aide d'un second banc de tirage à une vitesse V2 avant d'être bobinée à nouveau. Le ratio d'étirement V2/V1 permet de faire varier le diamètre. L'étirement permet d'affiner le diamètre de la fibre et d'améliorer ses propriétés mécaniques.

Le procédé 100 de fabrication d'une fibre multi-composante peut comporter **une étape de formation d'un tissu 140** à partir de la fibre multi-composante. L'étape de formation du tissu 140 à partir de la fibre multi-composante peut comporter ou non une étape de tissage ou de tricotage.

L'étape de formation du tissu 140 peut être réalisée à partir d'une seule fibre multi-composante, de plusieurs fibres multi-composantes ou encore d'une nappe de fibres multi-composantes. Le tissu formé pourra correspondre à un tissu non tissé ou à un tissu tissé en forme plate, tubulaire et en quelques autres motifs tridimensionnels. Comme pour les autres structures textiles, diverses propriétés peuvent être intégrées au maillage pour répondre aux objectifs de conception qui peuvent inclure une flexibilité accrue, une résistance accrue, une épaisseur réduite, une manipulation améliorée et une meilleure résistance mécanique.

Le procédé 100 de fabrication d'une fibre multi-composante peut comporter **une étape de mise en forme 150 du tissu.** Cette étape de mise en forme 150 permet de former un tissu qui pourra présenter une grande diversité de conformation grâce à la flexibilité de la fibre multi-composante comportant des polymères thermoplastiques.

Le procédé 100 de fabrication d'une fibre multi-composante comprend **une étape d'élimination 160** de l'au moins un polymère en surface de la fibre multi-composante. De préférence du polymère organique. L'étape d'élimination permet de générer une fibre multi-composante photocatalytique. Avantageusement, l'étape d'élimination comporte un traitement en surface de la fibre multi-composante.

De préférence, l'étape d'élimination de l'au moins un polymère, de préférence organique, en surface de la fibre multi-composante permet de générer une surface inorganique, de préférence majoritairement inorganique, au contact d'un support polymérique thermoplastique. Cela permet par exemple de former une écorce inorganique entourant un cœur polymérique thermoplastique dans une configuration de fibre cœur-écorce. La surface inorganique, de préférence majoritairement inorganique, générée peut comprendre la présence de polymère organique. Toutefois, le polymère organique restant sera minoritaire en poids dans la surface inorganique. En effet, la surface inorganique comporte de préférence au moins 50 % en poids de photocatalyseur et d'agent couplant.

Une étape d'élimination de l'au moins un polymère, de préférence organique, en surface peut comprendre un traitement thermique, un traitement chimique, un plasma, de préférence localisé, c'est-à-dire en surface de la fibre multi-composante. De préférence, le traitement permet d'éliminer au moins une partie du polymère organique du mélange actif sur une profondeur d'au moins 500 nm, de façon plus préférée au moins 1 µm, de façon encore plus préférée au moins 2 µm. De préférence, le traitement permet d'éliminer au moins une partie du polymère organique du mélange actif sur une profondeur d'au plus 50 µm, de façon plus préférée au plus 30 µm, de façon encore plus préférée au plus 20 µm. Par exemple, l'étape d'élimination permet de dégrader au moins une partie de l'au moins un polymère organique du mélange actif sur une profondeur comprise de 500 nm à 50 µm, de préférence de 1 µm à 30 µm, et de façon plus préférée de 2 µm à 20 µm.

Dans le mode de réalisation particulier illustré en lien avec la figure 2B, la matrice sacrificielle peut être éliminée antérieurement à l'étape d'élimination 160 également selon un traitement chimique, thermique, ou encore plasma.

Par ailleurs, l'étape d'élimination de l'au moins un polymère, de préférence organique, en surface de la fibre multi-composante, peut être partielle ou totale. Une élimination partielle peut correspondre à une élimination limitée sur la profondeur de la fibre multi-composante du ou des polymères organiques.

Un traitement thermique peut être sélectionné parmi : un traitement par rayonnement UV (ultraviolet) ou IR (infrarouge), un chauffage par convection, un chauffage par conduction. De préférence, un traitement thermique comprend une calcination. Un traitement thermique est de préférence localisé en surface et de façon plus préférée sur une profondeur d'au moins 500 nm. Un traitement thermique peut être réalisé à une température comprise entre 350 °C et 550 °C. Un traitement thermique peut être appliqué sur une durée comprise entre 0,5 heure et 7 heures.

Un traitement chimique peut être sélectionné parmi un traitement au moyen d'une espèce réactive qui soit sous forme solide, liquide, ou gaz et qui va permettre d'éliminer le polymère du mélange actif, par exemple, un traitement dans un liquide oxydant de type eau oxygénée.

Un traitement par plasma comportera généralement une ionisation de gaz. Le traitement par plasma pourra par exemple comporter une application de champs électriques ou magnétiques à travers un gaz de façon à former un plasma capable d'oxyder la surface de la fibre multi composante.

Une élimination du polymère de préférence organique en surface permet d'augmenter le taux de photocatalyseur en surface ce qui permet d'augmenter l'efficacité de la photocatalyse et donc d'améliorer grandement les propriétés de photocatalyse de la fibre-multi-composante.

Selon un autre aspect, l'invention concerne une **fibre multi-composante photocatalytique.**

Une fibre multi-composante photocatalytique selon l'invention est susceptible d'être obtenue par le procédé selon l'invention. De façon préférée, elle est directement obtenue par le procédé selon l'invention.

Une fibre multi-composante comprend un support comprenant au moins un polymère thermoplastique et une surface inorganique, de préférence une surface majoritairement inorganique.

Dans un mode de réalisation particulier, une fibre multi-composante photocatalytique selon l'invention comprend un cœur polymérique thermoplastique et une écorce inorganique.

Dans un mode de réalisation optionnel, la fibre multi-composante photocatalytique peut comprendre, de préférence entre le cœur et l'écorce, au moins une écorce intermédiaire. Une écorce intermédiaire peut comprendre un autre polymère thermoplastique, des matières inorganiques (ex :silice), et/ou des agents couplants. Une écorce intermédiaire peut par exemple permettre de protéger le cœur, d'augmenter la durée de vie de la fibre multi-composante. La fibre multi-composante peut également comprendre une matrice sacrificielle.

Le cœur polymérique thermoplastique de la fibre multi-composante comporte des polymères thermoplastiques. Le cœur polymérique de la fibre multi-composante peut comprendre des additifs. L'épaisseur du cœur de la fibre multi-composante peut être comprise entre 1 µm et 150 µm.

L'écorce de la fibre multi-composante comporte un réseau d'agent couplant résistant à l'oxydation et de photocatalyseur. De préférence, un réseau de silice et de titane. L'épaisseur en surface de photocatalyseur est de préférence comprise entre 300 nm et 20 µm. Avantageusement, l'écorce de la fibre multi-composante est au moins à 50 % inorganique, de préférence au moins 60 %, de manière plus préférée au moins 70 % et de manière encore plus préférée au moins 80 %. L'écorce de la fibre multi-composante peut être à moins de 100 % inorganique, par exemple de préférence à moins de 95%. Avantageusement, l'écorce inorganique présente au moins 5 % en poids de photocatalyseur, de préférence au moins 10 % en poids de photocatalyseur, de manière préférée au moins 15 % en poids de photocatalyseur, de manière plus préférée au moins 20 % en poids de photocatalyseur et de manière encore plus préférée au moins 25 %. Par exemple, l'écorce inorganique présente moins de 95 % en poids de photocatalyseur, de préférence moins de 90 % en poids de photocatalyseur et de manière plus préférée moins de 85 % en poids de photocatalyseur, de préférence après élimination du polymère organique du mélange actif. L'épaisseur de l'écorce de la fibre multi-composante peut être comprise entre 300nm et 20µm. La fibre multi-composante photocatalytique selon l'invention présente une épaisseur, de préférence un diamètre, inférieur(e) ou égal(e) à 150 µm, de préférence inférieur ou égal à 100 µm, de manière plus préférée inférieur ou égal à 50 µm, de manière encore plus préférée inférieur ou égal à 10 µm. Par exemple, la fibre multi-composante photocatalytique présente une épaisseur, de préférence un diamètre, d'au moins 1 µm.

De préférence, la fibre multi-composante présente une épaisseur de surface majoritairement inorganique, supérieure ou égale à 500 nm.

La fibre multi-composante présente une surface. De préférence la surface de la fibre multi-composante est homogène en composition (MEB/EDX pour Energy Dispersive X-ray en terminologie anglo-saxone). De préférence, après traitement, la fibre multi-composante est poreuse avec une surface spécifique d'au moins 10 m²/g (mesurée par analyse BET). La fibre multi-composante peut présenter une surface spécifique d'au plus 500 m²/g. De préférence la fibre multi-composante peut présenter une surface spécifique comprise entre 10 m²/g et 500m²/g.

Selon un autre aspect, l'invention concerne **un textile** comportant au moins une fibre multi-composante photocatalytique selon l'invention.

Un textile selon l'invention peut correspondre à un textile utilisable dans tous domaines comme par exemple le domaine vestimentaire, décoratif, ou industriel. Il peut être utilisé dans des procédés de traitement de l'eau et/ou de l'air, notamment via photocatalyse pour la purification de l'eau et/ou de l'air, ou pour ses propriétés anti-bactériennes et auto-nettoyantes. Un tel textile pourra être impliqué dans la destruction de composés organiques de préférence volatils.

Avantageusement, un textile selon l'invention est apte à être déformé afin de présenter différentes géométries. Ainsi un textile selon l'invention est flexible en particulier avant l'étape d'élimination de l'au moins un polymère de préférence organique en surface. La déformation peut être par pliage, tordage, torsadé sans que le textile ne casse ou perde en efficacité de photocatalyse. Ainsi, un filtre selon la présente invention présente une efficacité et une capacité photocatalytique augmentée.

Selon un autre aspect, l'invention concerne **un filtre** comportant au moins un textile selon l'invention.

Selon un autre aspect, l'invention concerne **un épurateur de fluide photocatalytique** comprenant au moins un filtre selon l'invention. Par fluide il peut par exemple s'agir d'air ou d'eau, de préférence d'air. De préférence le filtre est agencé entre une entrée de fluide et une sortie de fluide de l'épurateur.

Un épurateur peut également comprendre un système de ventilation.

Le système de ventilation peut être agencé de façon à véhiculer un fluide depuis une entrée de l'épurateur à une sortie de l'épurateur. Un système de ventilation est en particulier agencé de sorte à générer un flux d'air passant au travers du filtre selon l'invention. Un système de ventilation peut par exemple comprendre au moins un ventilateur doté d'une ou plusieurs hélices, pales et/ou turbines pouvant être agencées selon différentes positions. De préférence, le système de ventilation est agencé de façon à ce qu'un flux d'air, de préférence filtré, sortant de l'épurateur comporte un taux de COV, d'allergène, de polluant et/ou de pathogène réduit par rapport à un air entrant dans l'épurateur. Avantageusement, la vitesse du système de ventilation peut être adaptée. Ainsi différents niveaux de puissance de l'épurateur peuvent être définis. Ceci permet en cas de forte pollution par exemple d'augmenter la puissance de l'épurateur sans que celui-ci fonctionne en continu à puissance maximale.

Un épurateur peut également comprendre un système d'illumination.

Un système d'illumination comprend de préférence un système d'illumination ultraviolet apte à illuminer l'au moins un filtre selon l'invention. De préférence la longueur d'onde du système d'illumination est comprise dans la ou les gammes des U.V.A, des U.V. B et/ou des U.V. C soit de 100 à 400 nm. Avantageusement, le système d'illumination peut être centré sur une ou plusieurs bandes, par exemple de 100 nm à 280 nm, de 280 nm à 320 nm, de 320 nm 400 nm ou tout autres bandes permettant de centrer les U.V.A et les U.V.B ou les U.V.B et les U.V.C ou toutes autres combinaisons entre U.V.A, U.V.B, U.V.C. Le rayonnement UV permet d'activer le photocatalyseur. Le système d'illumination peut par exemple comprendre une lampe U.V.

Un épurateur peut comprendre un ou plusieurs capteurs. Par exemple, il peut s'agir de capteur de pression, de capteur de pollution, de capteur de température, de capteur de détection de particules, de capteur de concentration, de capteur de consommation, et de capteur d'obstruction.

Un épurateur peut comprendre un module de traitement configuré pour réguler la vitesse du système de ventilation et/ou l'intensité du système d'illumination.

Un épurateur peut comprendre un module de communication configuré pour communiquer entre le ou les capteurs et le système de ventilation et/ou d'illumination au travers d'un réseau de communication. Le module de communication peut par exemple être configuré pour émettre un message contenant au moins une indication de la différence de pression mesurée, du niveau d'obstruction du filtre, une indication d'un niveau de pollution, une indication d'un niveau de dépollution, un type de particule détectée, une élévation anormale de la température au sein de l'épurateur, et/ou une consommation anormale en courant de l'épurateur.

### EXEMPLES

### Composant

Polymère thermoplastique du mélange support : polypropylène
Polymère organique du mélange actif : polypropylène
Photocatalyseur : TiO₂ 40 %, sous forme cristalline : mélange d'anatase et de rutile
Agent couplant : polysiloxane

### Compoundage

Le compoundage est réalisé à l'aide d'un co-malaxeur disposant de capacité de mélange. Il peut s'agir d'un co-malaxeur mono-vis animé d'un mouvement de rotation et de translation ou d'un co-malaxeur bi-vis.

Le co-malaxeur peut comprendre plusieurs doseurs (gravimétriques et/ou volumétrique) et une ou plusieurs zones de chauffe.

Les polymères du mélange actif, l'agent couplant et éventuellement des additifs sont introduits dans le co-malaxeur à l'aide d'une trémie, et un mélange à sec est réalisé. Le photocatalyseur est introduit à l'aide du doseur gravimétrique et d'une alimentation transversale afin d'assurer l'incorporation du photocatalyseur à l'état fondu.

Un deuxième doseur gravimétrique sera rempli avec le mélange actif.

Le mélange support est également produit grâce à une extrudeuse mono ou bi-vis.

Le mélange est réalisé à une température comprise entre 150 et 300 °C.

### Filage

A l'issu du mélange, une étape de filage est réalisée à l'aide d'une bande de transport et de bobine(s). Les vitesses de chaque extrudeuse permettent de faire varier le ratio polymère du mélange support/polymère du mélange actif.

La caractérisation mécanique des fibres est effectuée selon la norme ISO 5079.

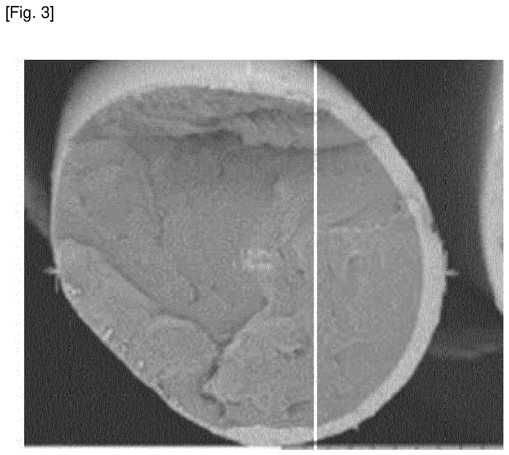

Comme cela est présenté à la **figure 3,** les fibres multi-composante photocatalytique selon l'invention sont beaucoup plus homogènes en composition, plus chargées en photocatalyseur, et présentent peu d'agrégats (MEB). Ainsi, il est possible d'apporter plus de matière active même avec des taux de gaine supérieur à 25%. Par ailleurs, leur diamètre diminue avec la présence d'une étape d'étirement tout en gardant de bonnes propriétés mécaniques. En effet, les fibres sont plus flexibles et plus résistantes tout en présentant un diamètre inférieur à 150 µm.

En outre, lorsque le procédé de fabrication comporte une étape préalable de greffage du photocatalyseur avec l'agent couplant les ratio d'étirement sont optimisés (8 contre 6) et les diamètres sont également réduits.

La fibre multi-composante photocatalytique selon l'invention présente des caractéristiques mécaniques améliorées. La fibre multi-composante présente une flexibilité optimisée ainsi qu'une malléabilité améliorée.

**[Tableau 1]**

| | **Fibre multi-composante-F3** | **Fibre multi-composante-F4** | **Fibre multi-composante-F16** |
|---|---|---|---|
| Emod (MPa) | 4608 | 3923 | 1930 |
| Titre (tex) | 20 | 10 | 14 |
| F_{H} (N) | 8,2 | 4,1 | 2,5 |
| Déformabilité (%) | 113,8 | 29,6 | 26,3 |
| σ_{H} (MPa) | 452,8 | 456,0 | 199,9 |
| Taux de gaine (%) | 13 | 13 | 26 |
| Charge TiO₂ (%en poids du mélange actif) avant calcination | 40 | 40 | 40 |
| Agent couplant (% en poids du mélange actif) | 10 | 10 | 10 |

L'invention peut faire l'objet de nombreuses variantes et applications autres que celles décrites ci-dessus. En particulier, sauf indication contraire, les différentes caractéristiques structurelles et fonctionnelles de chacune des mises en œuvre décrite ci-dessus ne doivent pas être considérées comme combinées et/ou étroitement et/ou inextricablement liées les unes aux autres, mais au contraire comme de simples juxtapositions. En outre, les caractéristiques structurelles et/ou fonctionnelles des différents modes de réalisation décrits ci-dessus peuvent faire l'objet en tout ou partie de toute juxtaposition différente ou de toute combinaison différente.

## Revendications

1. Procédé (100) de fabrication d'une fibre multi-composante photocatalytique comportant les étapes suivantes :
- Fournir un mélange support (110), ledit mélange support comportant au moins un polymère thermoplastique ou un précurseur de polymère thermoplastique ;
- Fournir d'un mélange actif (120), ledit mélange actif comportant :
∘ au moins un polymère organique ou un précurseur de polymère organique,
∘ au moins un photocatalyseur à une concentration d'au moins 10 % en poids par rapport au poids de mélange actif,
∘ au moins un agent couplant résistant à l'oxydation, de préférence un silane ; ou un précurseur d'agent couplant résistant à l'oxydation,
- Filage (130) d'une fibre multi-composante à partir des mélanges support et actif ;
- Elimination (160) de l'au moins un polymère organique en surface de la fibre multi-composante de façon à générer une fibre multi-composante photocatalytique.

2. Procédé (100) de fabrication d'une fibre multi-composante photocatalytique selon la revendication 1, **caractérisé en ce que** l'élimination de l'au moins un polymère organique en surface de la fibre multi-composante comprend une calcination de surface de façon à générer une surface inorganique au contact d'un support polymérique thermoplastique.

3. Procédé (100) de fabrication d'une fibre multi-composante photocatalytique selon la revendication 1 ou 2, **caractérisé en ce que** le ou les polymères thermoplastiques du mélange support sont sélectionnés parmi des polymères thermoplastiques présentant une température de fusion comprise entre 100 °C et 350 °C.

4. Procédé (100) de fabrication d'une fibre multi-composante photocatalytique selon l'une des revendications précédentes, **caractérisé en ce qu'**il comporte une extrusion et/ou une coextrusion du mélange support et/ou du mélange actif.

5. Procédé (100) de fabrication d'une fibre multi-composante photocatalytique selon l'une des revendications précédentes, **caractérisé en ce que** lors du filage la fibre multi-composante présente un taux de gaine compris entre 5 % et 50 % calculé selon la formule 4e * *(D - e*)/*D*².

6. Fibre multi-composante photocatalytique comportant :
- Un mélange support correspondant à un support polymérique thermoplastique comportant au moins un polymère thermoplastique, et
- Un mélange actif correspondant à une surface inorganique la surface inorganique comportant :
- un réseau d'agent couplant résistant à l'oxydation et
- de photocatalyseur,
la fibre multi-composante photocatalytique combinant le mélange support et le mélange actif filé.

7. Fibre multi-composante photocatalytique selon la revendication 6, **caractérisée en ce qu'**elle présente un diamètre inférieur ou égal à 150 µm.

8. Fibre multi-composante photocatalytique selon la revendication 6 ou 7, **caractérisée en ce qu'**elle présente une surface inorganique d'une épaisseur d'au moins 500 nm.

9. Textile comportant au moins une fibre multi-composante photocatalytique selon l'une des revendications 6 à 8.

10. Filtre comportant au moins un textile selon la revendication 9.

11. Epurateur d'air photocatalytique doté d'au moins un filtre selon la revendication 10, d'un système de ventilation et d'un système d'illumination ultraviolet apte à illuminer l'au moins un filtre ; ledit ventilateur étant agencé de façon à véhiculer de l'air depuis une entrée du système d'épuration à une sortie du système d'épuration à travers le filtre.

## Patentansprüche

1. Verfahren (100) zur Herstellung einer photokatalytischen Mehrkomponentenfaser, das die folgenden Schritte aufweist:
- Bereitstellen eines Trägergemischs (110), wobei das Trägergemisch mindestens ein thermoplastisches Polymer oder einen Vorläufer eines thermoplastischen Polymers umfasst;
- Bereitstellen eines aktiven Gemischs (120), wobei das aktive Gemisch aufweist:
∘ mindestens ein organisches Polymer oder einen Vorläufer eines organischen Polymers,
∘ mindestens einen Photokatalysator in einer Konzentration von mindestens 10 Gew.-%, bezogen auf das Gewicht des aktiven Gemischs,
∘ mindestens ein oxidationsbeständiges Kupplungsmittel, vorzugsweise ein Silan; oder einen Vorläufer eines oxidationsbeständigen Kupplungsmittels,
- Spinnen (130) einer Mehrkomponentenfaser aus dem Träger- und aktiven Gemisch;
- Entfernen (160) des mindestens einen organischen Polymers auf der Oberfläche der Mehrkomponentenfaser, um eine photokatalytische Mehrkomponentenfaser zu erzeugen.

2. Verfahren (100) zur Herstellung einer photokatalytischen Mehrkomponentenfaser nach Anspruch 1, **dadurch gekennzeichnet, dass** die Entfernung des mindestens einen organischen Polymers auf der Oberfläche der Mehrkomponentenfaser ein Kalzinieren der Oberfläche derart umfasst, dass eine anorganische Oberfläche in Kontakt mit einem thermoplastischen Polymerträger erzeugt wird.

3. Verfahren (100) zur Herstellung einer photokatalytischen Mehrkomponentenfaser nach Anspruch 1 oder 2, **dadurch gekennzeichnet, dass** das oder die thermoplastischen Polymere des Trägergemischs aus thermoplastischen Polymeren ausgewählt sind, die eine Schmelztemperatur zwischen 100°C und 350°C aufweisen.

4. Verfahren (100) zur Herstellung einer photokatalytischen Mehrkomponentenfaser nach einem der vorstehenden Ansprüche, **dadurch gekennzeichnet, dass** es eine Extrusion und/oder eine Coextrusion des Trägergemischs und/oder des aktiven Gemischs aufweist.

5. Verfahren (100) zur Herstellung einer photokatalytischen Mehrkomponentenfaser nach einem der vorstehenden Ansprüche, **dadurch gekennzeichnet, dass** die Mehrkomponentenfaser beim Spinnen einen Mantelanteil zwischen 5% und 50% aufweist, berechnet nach der Formel 4e * *(D - e*)/*D*².

6. Photokatalytische Mehrkomponentenfaser, aufweisend:
- ein Trägergemisch, das einem thermoplastischen Polymergemisch entspricht, das mindestens ein thermoplastisches Polymer aufweist, und
- ein aktives Gemisch, das einer anorganischen Oberfläche entspricht,
wobei die anorganische Oberfläche aufweist:
- ein oxidationsbeständiges Kupplungsmittelnetzwerk und
- Photokatalysator,
wobei die photokatalytische Mehrkomponentenfaser das Trägergemisch und das gesponnene aktive Gemisch kombiniert.

7. Photokatalytische Mehrkomponentenfaser nach Anspruch 6, **dadurch gekennzeichnet, dass** sie einen Durchmesser von weniger als oder gleich 150 µm aufweist.

8. Photokatalytische Mehrkomponentenfaser nach Anspruch 6 oder 7, **dadurch gekennzeichnet, dass** sie eine anorganische Oberfläche mit einer Dicke von mindestens 500 nm aufweist.

9. Textil mit mindestens einer photokatalytischen Mehrkomponentenfaser nach einem der Ansprüche 6 bis 8.

10. Filter, der mindestens ein Textil nach Anspruch 9 aufweist.

11. Photokatalytischer Luftreiniger, der mit mindestens einem Filter nach Anspruch 10, einem Belüftungssystem und einem UV-Beleuchtungssystem versehen ist, das imstande ist, den mindestens einen Filter zu beleuchten; wobei der Lüfter so eingerichtet ist, dass die Luft von einem Einlass des Reinigungssystems zu einem Auslass des Reinigungssystems durch den Filter befördert wird.

## Claims

1. **A method** (100) for manufacturing a photocatalytic multi-component fiber including the following steps:
- Providing a support mixture (110), said support mixture including at least one thermoplastic polymer or a thermoplastic polymer precursor;
- Providing an active mixture (120), said active mixture including:
∘ at least one organic polymer or one organic polymer precursor,
∘ at least one photocatalyst at a concentration of at least 10% by weight relative to the weight of active mixture,
∘ at least one coupling agent resistant to oxidation, preferably a silane; or a coupling agent precursor resistant to oxidation,
- Spinning (130) a multi-component fiber from support and active mixtures;
- Eliminating (160) the at least one organic polymer on the surface of the multi-component fiber so as to generate a photocatalytic multi-component fiber.

2. The method (100) for manufacturing a photocatalytic multi-component fiber according to claim 1, **characterized in that** the elimination of the at least one organic polymer on the surface of the multi-component fiber comprises a surface calcination so as to generate an inorganic surface in contact with a thermoplastic polymer support.

3. The method (100) for manufacturing a photocatalytic multi-component fiber according to claim 1 or 2, **characterized in that** the thermoplastic polymer(s) of the support mixture are selected from thermoplastic polymers having a melting temperature comprised between 100°C and 350°C.

4. The method (100) for manufacturing a photocatalytic multi-component fiber according to one of the preceding claims, **characterized in that** it includes an extrusion and/or a coextrusion of the support mixture and/or the active mixture.

5. The method (100) for manufacturing a photocatalytic multi-component fiber according to one of the preceding claims, **characterized in that** during spinning the multi-component fiber has a sheathing rate comprised between 5% and 50% calculated according to the formula 4e * *(D - e*)/*D*².

6. A photocatalytic multi-component fiber including
- A support mixture corresponding to a thermoplastic polymer support including at least a thermoplastic polymer, and
- A active mixture corresponding to an inorganic surface; the inorganic surface including a network of coupling agent, which is resistant to oxidation, and photocatalyst, the photocatalytic multi-component fiber combining the support mixture and the active mixture spun.

7. The photocatalytic multi-component fiber according to claim 6, **characterized in that** it has a diameter less than or equal to 150 µm.

8. The photocatalytic multi-component fiber according to claim 6 or 7, **characterized in that** it has an inorganic surface with a thickness of at least 500 nm.

9. A textile including at least one photocatalytic multi-component fiber according to one of claims 6 to 8.

10. A filter including at least one textile according to claim 9.

11. A photocatalytic air purifier provided with at least one filter according to claim 10, a ventilation system and an ultraviolet illumination system capable of illuminating the at least one filter; said ventilator being arranged to convey air from an inlet of the purification system to an outlet of the purification system through the filter.
